# EUROPÄISCHE PATENTSCHRIFT

(11) **EP 4 208 877 B1**
(45) Veröffentlichungstag und Bekanntmachung des Hinweises auf die Patenterteilung: **13.12.2023**
(21) Anmeldenummer: 22783335.7
(22) Anmeldetag: 19.09.2022
(51) Int. Cl.: G16H 40/20, G16H 40/63, A61B 90/98, A61B 34/20

(54) **SYSTEM UND VERFAHREN ZUR ÜBERWACHUNG ZUMINDEST EINES MEDIZINPRODUKTS**
SYSTEM AND METHOD FOR MONITORING AT LEAST ONE MEDICAL DEVICE
SYSTÈME ET PROCÉDÉ DE SURVEILLANCE D'AU MOINS UN DISPOSITIF MÉDICAL

(30) Priorität: 20.09.2021 DE 102021124194
(43) Veröffentlichungstag der Anmeldung: 12.07.2023
(73) Patentinhaber: Aesculap AG, 78532 Tuttlingen (DE)
(72) Erfinder: LENZENHUBER, Frederick, 78532 Tuttlingen (DE); HÖGERLE, Roland-Alois, 78532 Tuttlingen (DE)
(74) Vertreter: Winter, Brandl - Partnerschaft mbB
(86) Internationale Anmeldenummer: PCT/EP2022/075973
(87) Internationale Veröffentlichungsnummer: WO 2023/041785

(56) Entgegenhaltungen:
- EP-B1- 3 207 491
- US-A1- 2019 151 044
- US-A1- 2020 043 603

## Beschreibung

### Technisches Gebiet

Die vorliegende Erfindung bezieht sich auf ein System zur Überwachung zumindest eines Medizinprodukts gemäß dem Oberbegriff des Anspruchs 1, insbesondere auf ein System zur Überwachung zumindest eines, insbesondere wiederverwendbaren, Medizinprodukts, insbesondere eines chirurgischen Instruments, und/oder eines chirurgischen Brancheninstrument, mit zumindest zwei miteinander in einem Überwachungsnetzwerk datenleitend, insbesondere kabellos, verbundenen oder verbindbaren Überwachungsvorrichtungen, die jeweils ein Erkennungsgerät aufweisen, das ausgebildet ist, zu erkennen, ob sich in einem sich um die jeweilige Überwachungsvorrichtung erstreckenden Erkennungsbereich ein Transponder des Medizinprodukts befindet, wobei die zumindest zwei Überwachungsvorrichtungen derart angeordnet oder anordenbar sind, dass sich die Erkennungsbereiche der zumindest zwei Überwachungsvorrichtungen überlappen, und einer mit den zumindest zwei Überwachungsvorrichtungen über das Überwachungsnetzwerk datenleitend, insbesondere kabellos, verbundenen oder verbindbaren Datenverarbeitungsvorrichtung, die ausgebildet ist, Zeitpunkte von Erkennungen des Transponders durch die Überwachungsvorrichtungen speichern zu können. Die vorliegende Erfindung bezieht sich des Weiteren auf ein Verfahren zur Überwachung zumindest eines Medizinprodukts in einer Operationsumgebung.

### Stand der Technik

Ein gattungsgemäßes System zur Überwachung eines Medizinprodukts wird beispielsweise in der US 10 709 521 B2 offenbart. Bei dem System gemäß US 10 709 521 B2 sind Medizinprodukte jeweils mit zwei Transpondern ausgestattet. Ein Identifikationstransponder ist ausgebildet, als Reaktion auf ein eingehendes Signal ein Identifikationssignal zurückzusenden. Ein einfacher Transponder ist ausgebildet, als Reaktion auf ein eingehendes Signal ein einfaches Signal ohne Identifikationsinformationen zurückzusenden. Nach Einsatz bei einer Behandlung werden die Medizinprodukte gemäß US 10 709 521 B2 in abgeschirmte Behälter gegeben. In Laufe der Behandlung wird in bestimmten Phasen ein Signal ausgesendet, um Reaktionen von Identifikationstranspondern hervorzurufen, und in einer bestimmten Phase ein Signal ausgesendet, um Reaktionen von einfachen Transpondern hervorzurufen. Werden in den Phasen Reaktionen von Transpondern registriert, bedeutet das, dass Medizinprodukte noch nicht in die abgeschirmten Behälter gegeben wurden und beispielsweise bei einer Operation noch innerhalb einer behandelten Person sind.

Problem des Systems gemäß US 10 709 521 B2 ist, dass eine Überwachung eines einzelnen Medizinprodukts nur unzureichend möglich ist.

Die US 2019 / 0 151 044 A1 offenbart ein Registrieren von medizinischen Instrumenten mittels RFID-Antennen während klinischer Behandlungen.

Weitere Systeme zum Registrieren medizinischer Instrumente mittels RFID-Antennen werden beispielsweise in EP 3 207 491 B1 und US 2020 / 0 043 603 A1 offenbart.

### Zusammenfassung der Offenbarung

Aufgabe der vorliegenden Offenbarung ist es deshalb, ein System zur Überwachung zumindest eines Medizinprodukts bereitzustellen, das eine verbesserte Überwachung, insbesondere bezüglich verschiedener Einsatzund/oder Lagerbereiche, eines Medizinprodukts ermöglicht.

Diese Aufgabe wird durch Merkmale des Anspruchs 1 gelöst. Vorteilhafte Weiterbildungen sind Gegenstand der Unteransprüche.

Ein offenbarungsgemäßes System zur Überwachung zumindest eines Medizinprodukts weist zumindest zwei Überwachungsvorrichtungen auf. Die zumindest zwei Überwachungsvorrichtungen sind miteinander in einem Überwachungsnetzwerk datenleitend, kabellos bzw. nicht kabelgebunden, insbesondere per Funk, oder kabelgebunden, verbundenen oder verbindbar. Insbesondere können die zumindest zwei Überwachungsvorrichtungen auch stromleitend miteinander verbunden oder verbindbar sein. Die zumindest zwei Überwachungsvorrichtungen weisen jeweils ein Erkennungsgerät auf, das ausgebildet ist, erkennen zu können, ob sich in einem sich um die jeweilige Überwachungsvorrichtung erstreckenden Erkennungsbereich ein Transponder des Medizinprodukts befindet. Die zumindest zwei Überwachungsvorrichtungen sind derart angeordnet oder anordenbar, dass sich die Erkennungsbereiche der zumindest zwei Überwachungsvorrichtungen überlappen, das heißt, dass es Punkte oder Bereiche in der Umgebung der zumindest zwei Überwachungsvorrichtungen geben kann, die sowohl in dem Erkennungsbereich der einen der zumindest zwei Überwachungsvorrichtungen, als auch in dem Erkennungsbereich der anderen der zumindest zwei Überwachungsvorrichtungen liegen. Das offenbarungsgemäße System weist eine Datenverarbeitungsvorrichtung auf, die mit den zumindest zwei Überwachungsvorrichtungen über das Überwachungsnetzwerk datenleitend, kabellos bzw. nicht kabelgebunden, insbesondere per Funk, oder kabelgebunden, verbunden oder verbindbar ist. Insbesondere kann die Datenverarbeitungsvorrichtung mit den zumindest zwei Überwachungsvorrichtungen auch stromleitend verbunden oder verbindbar sein. Die Datenverarbeitungsvorrichtung ist ausgebildet, Zeitpunkte von Erkennungen des Transponders durch die Überwachungsvorrichtungen speichern zu können. Das heißt, dass die Datenverarbeitungsvorrichtung derart ausgebildet ist, jedes Mal, wenn eine der zumindest zwei Überwachungsvorrichtungen ein Signal zur Erkennung eines Transponders aussendet und ein entsprechendes Antwortsignal von dem Transponder empfängt, einen Zeitstempel zu diesem Vorgang zu speichern. Insbesondere speichert die Datenverarbeitungsvorrichtung in dem Zeitstempel eine Identifikation der Überwachungsvorrichtung, eine Identifikation des Transponders und einen Zeitpunkt des Empfangs des Antwortsignals.

Die Datenverarbeitungsvorrichtung ist offenbarungsgemäß ausgebildet, Zeitdauern, in welchen sich der Transponder ausschließlich in einem der Erkennungsbereiche der zumindest zwei Überwachungsvorrichtungen befindet, anhand gespeicherter Zeitpunkte zu berechnen bzw. berechnen zu können. Das heißt, die Datenverarbeitungsvorrichtung ist insbesondere ausgebildet, die Zeitpunkte bzw. Zeitstempel einzelner Erkennungen bzw. Erkennungsvorgängen des Transponders bzw. eines bestimmten Transponders durch die zumindest zwei Überwachungsvorrichtungen abzugleichen bzw. abgleichen zu können und diejenigen Zeitpunkte bzw. Zeitstempel zu separieren bzw. separieren zu können, in welchen der Transponder bzw. ein bestimmter Transponder nur von einer der zumindest zwei Überwachungsvorrichtungen erkannt wird. Die Datenverarbeitungsvorrichtung ist des Weiteren insbesondere ausgebildet, aus diesen separierten Zeitpunkten bzw. Zeitstempeln jeweils zeitlich aufeinanderfolgende Zeitpunkte bzw. Zeitstempel auszusortieren bzw. aussortieren zu können, welche repräsentativ sind für Zeitdauern, in denen sich der Transponder bzw. ein bestimmter Transponder nur in dem Erkennungsbereich einer der zumindest zwei Überwachungsvorrichtungen befindet. Die Datenverarbeitungsvorrichtung ist insbesondere ausgebildet, die Zeitdauer aufeinander folgender Zeitpunkte bzw. Zeitstempel jeweils durch Subtraktion des ältesten Zeitpunkts bzw. Zeitstempels von dem jüngsten Zeitpunkt bzw. Zeitstempels zu berechnen bzw. berechnen zu können.

Zusätzlich oder alternativ ist die Datenverarbeitungsvorrichtung ausgebildet, Reihenfolgen, in welchen die zumindest zwei Überwachungsvorrichtungen den Transponder in den Erkennungsbereichen erkennen, registrieren zu können. Insbesondere kann die Datenverarbeitungsvorrichtung ausgebildet sein, durch Abgleich von Daten der zumindest zwei Überwachungsvorrichtungen erkennen zu können, ob sich der Transponder im Rahmen eines Ereignisses einer ersten Art ausschließlich in einem einzigen der Erkennungsbereiche oder im Rahmen eines Ereignisses einer zweiten Art zugleich in mehreren der Erkennungsbereiche befindet, und zumindest ein Ereignis der ersten Art und zumindest ein Ereignis der zweiten Art in einer Liste speichern zu können. Insbesondere können die Ereignisse derart mit zugehörigen Zeitdaten abgespeichert werden, dass die Ereignisse in einer zeitlichen Reihenfolge geordnet sind.

Durch Berechnung von Zeitdauern und/oder Registrierung von Reihenfolgen ist es in vorteilhafter Weise möglich, eine Überwachung eines Medizinprodukts kohärenter auszuführen. Durch Berechnung der Zeitdauern, in welchen sich der Transponder nur in einem Erkennungsbereich befindet, kann durch entsprechende Anordnung der Überwachungsvorrichtungen ein Aufenthaltsbereich des Transponders bzw. des Medizinprodukts, in welchem eine Überwachung besonders wichtig ist, exakter oder enger eingegrenzt werden.

Die Grenze des jeweiligen Erkennungsbereichs kann durch eine Reichweite einer eingesetzten Erkennungstechnologie, durch eine physikalische Grenze (zum Beispiel eine Wand eines Behälters) oder durch eine geometrische Definition (zum Beispiel Entfernung vom Erkennungsgerät) vorgegeben sein.

Das System weist einen Speicher auf, in welchem Soll-Zeitdauern und/oder Soll-Reihenfolgen gespeichert sind. Die Datenverarbeitungsvorrichtung ist ausgebildet, die berechneten Zeitdauern mit den entsprechenden Soll-Zeitdauern und/oder die registrierten Reihenfolgen mit den Soll-Reihenfolgen zu vergleichen und bei einem Überschreiten von Soll-Zeitdauern und/oder bei einem Abweichen von Soll-Reihenfolgen entsprechende Alarmsignale auszugeben.

Durch Vergleichen der berechneten Zeitdauern mit Soll-Zeitdauern kann eine Wahrscheinlichkeit für eine über eine vorbestimmte Lebensdauer hinausgehende Benutzung eines Medizinprodukts in vorteilhafter Weise verringert werden.

Gemäß einem Aspekt er Offenbarung kann das Medizinprodukt widerverwendbar und/oder ein chirurgisches Instrument sein. Das Medizinprodukt kann insbesondere ein nicht stromführendes chirurgisches Instrument bzw. ein chirurgisches Instrument ohne eine eigene Antriebseinheit sein.

Gemäß einem Aspekt der Offenbarung können die zumindest zwei Überwachungsvorrichtungen derart ausgebildet sein, dass die zugehörigen Erkennungsbereiche unterschiedlich groß sind.

Durch Vorsehen von Überwachungsvorrichtungen mit unterschiedlich großen Erkennungsbereichen kann eine Überwachung in vorteilhafter Weise an unterschiedlich eingeschränkte Aufenthaltsbereiche eines Medizinprodukts angepasst werden. Befindet sich ein Medizinprodukt zum Beispiel in einem Bereich eines Behälters, kann mit dem System gemäß dem Aspekt auf effiziente Weise durch Anbringen einer Überwachungsvorrichtung mit einem vergleichsweise kleinen Erkennungsbereich an dem Behälter erkannt werden, ob sich das Medizinprodukt innerhalb oder außerhalb des Behälters befindet.

Gemäß einem Aspekt der Offenbarung kann zumindest eines der zumindest zwei Erkennungsgeräte ausgebildet sein, erkennen zu können bzw. zu erkennen, ob sich der Transponder des Medizinprodukts in einem sich um die jeweilige Überwachungsvorrichtung erstreckenden und im Vergleich zum entsprechenden Erkennungsbereich kleineren Naherkennungsbereich befindet und/oder ausgebildet sein, erkennen zu können bzw. zu erkennen, ob sich der Transponder des Medizinprodukts in einem sich um die jeweilige Überwachungsvorrichtung erstreckenden und im Vergleich zum entsprechenden Erkennungsbereich größeren Fernerkennungsbereich befindet. Anders ausgedrückt kann zumindest eines der zumindest zwei Erkennungsgeräte ausgebildet sein, einen sich in der Umgebung des entsprechenden Erkennungsgeräts befindlichen unterschiedlichen Entfernungsbereichen zuordnen zu können. Insbesondere kann eines der zumindest zwei Erkennungsgeräte ausgebildet sein, eine Intensität von Signalen, die das Erkennungsgeräte zur Erkennung des Transponders aussendet bzw. aussenden kann, variieren zu können, insbesondere zyklisch variieren zu können bzw., insbesondere zyklisch, zu variieren.

Gemäß einem Aspekt der Offenbarung kann die Datenverarbeitungsvorrichtung ausgebildet sein, Zeitdauern, in welchen sich der Transponder in dem zumindest einen Naherkennungsbereich und/oder in dem zumindest einen Fernerkennungsbereich befindet, anhand der gespeicherten Zeitpunkte bzw. Zeitstempel der Erkennungen in dem Naherkennungsbereich und entsprechend der gespeicherten Zeitpunkte bzw. Zeitstempel der Erkennungen in dem Fernerkennungsbereich zu berechnen bzw. berechnen zu können.

Gemäß einem Aspekt der Offenbarung kann die Datenverarbeitungsvorrichtung ausgebildet ist, Zeitdauern, in welchen sich der Transponder nicht in dem zumindest einen Naherkennungsbereich und/oder nicht in dem zumindest einen Fernerkennungsbereich befindet, anhand der gespeicherten Zeitpunkte bzw. Zeitstempel der Erkennungen in dem Naherkennungsbereich und entsprechend der gespeicherten Zeitpunkte bzw. Zeitstempel der Erkennungen in dem Fernerkennungsbereich zu berechnen bzw. berechnen zu können.

Durch Vorsehen eines zusätzlichen Naherkennungsbereichs und/oder eines zusätzlichen Fernerkennungsbereichs und durch Berechnung entsprechender Zeitdauern kann eine räumliche Auflösung einer Überwachung in vorteilhafter Weise vergrößert werden.

Gemäß einem Aspekt der Offenbarung kann zumindest eines der Erkennungsgeräte ausgebildet sein, zur Erkennung des Transponders elektromagnetische Wellen, insbesondere Radiowellen, eines ersten Frequenzbandes, insbesondere mit einer Frequenz zwischen 860 MHz und 950 MHz oder mit einer Frequenz zwischen 2,45 GHz und 6 GHz, insbesondere zwischen 2,45 GHz und 5,8 GHz, aussenden zu können bzw. auszusenden und von dem Transponder empfangen zu können bzw. zu empfangen, und kann zumindest eines der Erkennungsgeräte ausgebildet sein, zur Erkennung des Transponders elektromagnetische Wellen, insbesondere Radiowellen, eines zweiten Frequenzbandes, insbesondere mit einer Frequenz von 13,56 MHz, aussenden zu können bzw. auszusenden und von dem Transponder empfangen zu können bzw. zu empfangen. Insbesondere kann eines der Erkennungsgeräte zum Betrieb in dem ersten Frequenzband ausgebildet sein und kann ein anderes Erkennungsgerät zum Betrieb in dem zweiten Frequenzband ausgebildet sein. Offenbarungsgemäß kann auch ein Erkennungsgerät ausgebildet sein, sowohl im ersten, als auch im zweiten Frequenzband betrieben zu werden. Insbesondere können mehrere Erkennungsgeräte vorgesehen sein, die jeweils zum Betrieb in einem jeweiligen Frequenzband ausgebildet sind.

Durch Abstimmung der Erkennungsgeräte auf das erste und/oder zweite Frequenzband kann das offenbarungsgemäße System in vorteilhafterweise auf unterschiedliche Umgebungsbedingungen und damit unterschiedliche Dämpfungen angepasst werden.

Gemäß einem Aspekt der Offenbarung kann zumindest eines der Erkennungsgeräte, insbesondere das zumindest eine zur Erkennung des Transponders in dem Fernerkennungsbereich ausgebildete Erkennungsgerät, ausgebildet sein, zur Erkennung des Transponders, insbesondere in dem entsprechenden Erkennungsbereich, elektromagnetische Wellen, insbesondere Radiowellen, gemäß einem ersten Übertragungsstandard, insbesondere einem Nahfeldkommunikationsstandard, aussenden zu können bzw. auszusenden und von dem Transponder empfangen zu können bzw. zu empfangen, und kann zumindest eines der Erkennungsgeräte, insbesondere das zumindest eine zur Erkennung des Transponders in dem Fernerkennungsbereich ausgebildete Erkennungsgerät, ausgebildet sein, zur Erkennung des Transponders, insbesondere in dem entsprechenden Fernerkennungsbereich, elektromagnetische Wellen, insbesondere Radiowellen, gemäß einem zweiten Übertragungsstandard, insbesondere einem RFID-Übertragungsstandards, aussenden zu können bzw. auszusenden und von dem Transponder empfangen zu können bzw. zu empfangen. Insbesondere kann eines der Erkennungsgeräte zum Betrieb in dem ersten Übertragungsstandard ausgebildet sein und kann ein anderes Erkennungsgerät zum Betrieb in dem zweiten Übertragungsstandard ausgebildet sein und somit insbesondere unterschiedlich große Erkennungsbereiche zu realisieren. Offenbarungsgemäß kann auch ein Erkennungsgerät, insbesondere das zumindest eine zur Erkennung des Transponders in dem Fernerkennungsbereich ausgebildete Erkennungsgerät, ausgebildet sein, sowohl im ersten, als auch im zweiten Übertragungsstandard betrieben zu werden. Insbesondere kann ein Erkennungsgerät ausgebildet sein, den bzw. einen Transponder in dem Erkennungsbereich anhand des ersten Übertragungsstandard erkennen zu können bzw. zu erkennen und in dem Naherkennungsbereich und/oder Fernerkennungsbereich anhand des zweiten Übertragungsstandards erkennen zu können bzw. zu erkennen.

Werden Erkennungsgeräte entsprechend bekannter Übertragungsstandards betrieben, ist es in vorteilhafterweise möglich, herkömmliche Lesegeräte in ein offenbarungsgemäßes System einzubinden. Beispielsweise kann ein Smartphone durch entsprechende Programmierung zur Erkennung eines Transponders mittels eines Nahfeldkommunikationsstandards in ein offenbarungsgemäßes Erkennungsgerät bzw. in eine offenbarungsgemäße Überwachungsvorrichtung umzuwandeln.

Gemäß einem Aspekt der Offenbarung kann das Überwachungsnetzwerk ausgebildet sein, gemäß einem dritten Übertragungsstandard, insbesondere einem WLAN-Standard nach IEEE 802.11 oder einem WPAN-Standard nach IEEE 802.15 wie beispielsweise Bluetooth^{®}, oder gemäß Bluetooth^{®} Low Energy (BLE) betrieben zu werden.

Wird das Überwachungsnetzwerk in einem Standard betrieben, der sich von den Standards zur Erkennung des Transponders unterscheidet, können gegenseitige Störungen in vorteilhafter Weise verhindert oder zumindest verringert werden.

Gemäß einem Aspekt der Offenbarung kann das Überwachungsnetzwerk teilweise oder vollständig vermascht sein. Insbesondere können mehrere Überwachungsvorrichtungen jeweils mit Datenverarbeitungseinheiten ausgestattet sein, welche jeweils die von der Datenverarbeitungsvorrichtung berechneten Zeitdauern von der Datenverarbeitungsvorrichtung empfangen und speichern können.

Durch eine vermaschte Ausbildung des Überwachungsnetzwerks kann bei Ausfall einer Überwachungsvorrichtung oder einer Verbindung durch Umleitung der Datenkommunikation die Datenkommunikation weiterhin ermöglicht werden.

Gemäß einem Aspekt der Offenbarung kann zumindest eine Überwachungsvorrichtung in einem Sterilgutbehälter angeordnet sein bzw. kann das offenbarungsgemäße System zumindest einen Sterilgutbehälter aufweisen, der mit einer Überwachungsvorrichtung ausgestattet ist.

Gemäß einem Aspekt der Offenbarung kann zumindest eine Überwachungsvorrichtung in einem Lagerungsbereich einer Operationsumgebung angeordnet sein, kann zumindest eine Überwachungsvorrichtung in der Operationsumgebung in einem Sterilbereich an einem Beistelltisch in der Umgebung eines Operationstisches angeordnet sein und kann zumindest eine Überwachungsvorrichtung in der Operationsumgebung in dem Sterilbereich an dem Operationstisch angeordnet sein. Der Lagerungsbereich ist insbesondere weiter von dem Operationstisch entfernt als der Beistelltisch. Der Sterilbereich ist insbesondere ein sich um den Operationstisch erstreckender Bereich, dessen Grenzen von dem Operationstisch mindestens einen Meter entfernt ist.

Durch die Anordnung der Überwachungsvorrichtungen in einem Sterilgutbehälter und/oder in Bereichen, die bei einer Benutzung eines Medizinprodukts durchquert werden, kann eine verbesserte, insbesondere lückenlose, Überwachung ermöglicht werden.

Gemäß einem Aspekt der Offenbarung kann ein offenbarungsgemäßes System ein Positionierungssystem aufweisen, das ausgebildet ist, relative Positionen der zumindest zwei Überwachungsvorrichtungen detektieren und/oder registrieren zu können. Insbesondere kann das Positionierungssystem Sensoren aufweisen, welche die Positionen der zumindest zwei Überwachungsvorrichtungen detektieren können. Alternativ oder zusätzlich können die Überwachungsvorrichtungen Schnittstellen zu einem globalen Navigationssatellitensystem wie zum Beispiel NAVSTAR GPS oder Galileo aufweisen, über welche die jeweilige Überwachungsvorrichtung ihre eigene Position ermitteln können, und können die Überwachungsvorrichtungen ausgebildet sein, ihre Position dem Positionierungssystem übermitteln zu können, welche ausgebildet sein kann, aus den Positionen der Überwachungsvorrichtungen relative Positionen der Überwachungsvorrichtungen berechnen und registrieren zu können. Alternativ oder zusätzlich kann das Positionierungssystem auch derart ausgebildet sein, dass relative Positionen der zumindest zwei Überwachungsvorrichtungen über ein Eingabegerät wie einen berührungsempfindlichen Bildschirm, eine Tastatur oder eine Maus eingegeben werden können und von dem Positionierungssystem registriert werden können.

Werden Relativpositionen der Überwachungsvorrichtungen berücksichtigt, kann auch dann, wenn die sich Erkennungsbereiche der Überwachungsvorrichtungen nicht überlappen, abgeschätzt werden, wo sich ein Medizinprodukt mit einer gewissen Wahrscheinlichkeit befindet und ob ein NichtErkennen eines Medizinprodukts kritisch ist oder nicht.

Die Offenbarung betrifft des Weiteren ein Verfahren zur Überwachung zumindest eines Medizinprodukts in einer Operationsumgebung (zum Beispiel in einem Operationssaal). In der Operationsumgebung sind zumindest zwei miteinander in einem Überwachungsnetzwerk datenleitend verbundene oder verbindbare Überwachungsvorrichtungen, derart angeordnet oder anordenbar, dass sich um die jeweilige Überwachungsvorrichtung erstreckende Erkennungsbereiche der zumindest zwei Überwachungsvorrichtungen überlappen. Das Medizinprodukt ist insbesondere wiederverwendbar und/oder ein chirurgisches Instrument. Das Verfahren weist folgende Schritte auf:
- Erkennen, ob sich in einem der Erkennungsbereiche ein Transponder des Medizinprodukts befindet,
- Speichern von Zeitpunkten von Erkennungen des Transponders, und
- Berechnen von Zeitdauern, in welchen sich der Transponder ausschließlich in einem der Erkennungsbereiche der zumindest zwei Überwachungsvorrichtungen befindet, Vergleichen der berechneten Zeitdauern mit entsprechenden Soll-Zeitdauern und Ausgeben entsprechender Alarmsignale bei einem Überschreiten von Soll-Zeitdauern und/oder
- Registrieren von Reihenfolgen, in welchen die zumindest zwei Überwachungsvorrichtungen den Transponder in den Erkennungsbereichen erkennen, Vergleichen der registrierten Reihenfolgen mit entsprechenden gespeicherten Soll-Reihenfolgen und Ausgeben entsprechender Alarmsignale bei einem Abweichen von Soll-Reihenfolgen.

Der Erkennen-Schritt wird insbesondere durch ein Erkennungsgerät der dem jeweiligen Erkennungsbereich zugehörigen Überwachungsvorrichtung ausgeführt. Die Grenze des jeweiligen Erkennungsbereichs kann durch eine Reichweite einer eingesetzten Erkennungstechnolgie, durch eine physikalische Grenze (zum Beispiel eine Wand eines Behällters) oder durch eine geometrische Definition (zum Beispiel Entfernung vom Erkennungsgerät) vorgegeben sein.

Der Speichern-Schritt und/oder der Berechnen-Schritt werden/wird insbesondere durch eine mit den zumindest zwei Überwachungsvorrichtungen über das Überwachungsnetzwerk datenleitend verbundene oder verbindbare Datenverarbeitungsvorrichtung ausgeführt.

### Kurzbeschreibung der Zeichnungen

Die vorliegende Offenbarung wird im Folgenden anhand eines bevorzugten Ausführungsbeispiels unter Bezugnahme auf die beigefügten Zeichnungen näher beschrieben. Es zeigen:
Fig. 1 eine perspektivische Ansicht eines Medizinprodukts in Form eines chirurgischen Mikromotorinstruments mit einem Transponder;
Fig. 2 eine perspektivische Ansicht eines Medizinprodukts in Form eines Nadelhalters mit einem Transponder;
Fig. 3 eine schematische Ansicht eines offenbarungsgemäßen Systems in einem Zustand, in welchem Medizinprodukte in Sterilgutbehältern auf einem Lagertisch abseits eines Sterilbereichs gelagert sind;
Fig. 4 eine schematische Ansicht des Systems gemäß Fig. 3 in einem Zustand, in welchem sich einer der Sterilgutbehälter außerhalb eines Lagerbereichs und teilweise außerhalb des Sterilbereichs befindet;
Fig. 5 eine schematische Ansicht des Systems gemäß Fig. 3 in einem Zustand, in welchem einer der Sterilgutbehälter auf einem Beistelltisch innerhalb des Sterilbereichs zwischengelagert ist und sich einer der Sterilgutbehälter außerhalb des Lagerbereichs und außerhalb des Sterilbereichs befindet,
Fig. 6 eine schematische Ansicht des Systems gemäß Fig. 3 in einem Zustand, in welchem ein Medizinprodukt, das dem sich außerhalb des Sterilbereichs befindlichen Sterilgutbehälter entnommen ist, alternativ außerhalb oder innerhalb des Sterilbereichs befindet;
Fig. 7 eine schematische Ansicht des Systems gemäß Fig. 3 in einem Zustand, in welchem eines der Medizinprodukte innerhalb des Sterilbereichs von dem Beistelltisch entfernt ist, sich aber außerhalb eines Operationsbereichs befindet;
Fig. 8 eine schematische Ansicht des Systems gemäß Fig. 3 in einem Zustand in welchem eines der Medizinprodukte innerhalb des Sterilbereichs von dem Beistelltisch entfernt ist und sich in der Nähe eines Operationsbereichs befindet;
Fig. 9 eine schematische Ansicht des Systems gemäß Fig. 3 in einem Zustand, in welchem sich eines der Medizinprodukte innerhalb eines Operationsbereichs befindet; und
Fig. 10 eine schematische Ansicht eines durch Überlappung mehrerer Erkennungsbereiche gebildeten Überwachungsbereichs.

### Detaillierte Beschreibung einer bevorzugten Ausführungsform

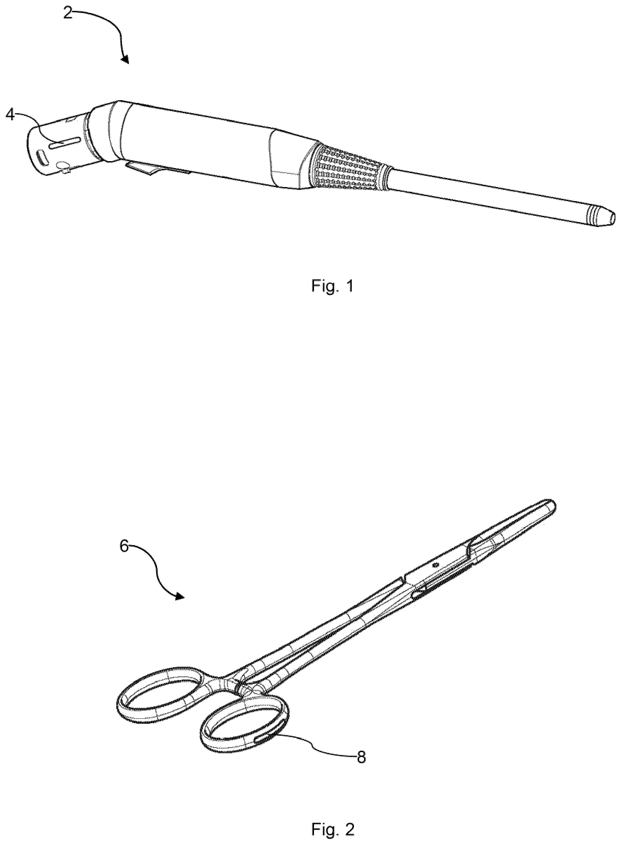

Fig. 1 zeigt eine perspektivische Ansicht eines Medizinprodukts in Form eines chirurgischen Mikromotorinstruments 2 mit einem Transponder 4. Der Transponder 4 ist an einem Anschluss an einem proximalen Ende des Mikromotorinstruments 2 angebracht.

Fig. 2 zeigt eine perspektivische Ansicht eines Medizinprodukts in Form eines Nadelhalters 6 mit einem Transponder 8. Der Transponder 8 ist an einem Ring einer Grifföffnung des Nadelhalters 6 angebracht.

Die Transponder 4 und 8 sind jeweils ausgebildet, bei einem Empfang von Signalen nach einem Nahkommunikationsstandard ein entsprechendes Antwortsignal auszusenden und bei einem Empfang von Signalen nach einem (anderen) RFID-Standard ein entsprechendes (anderes) Antwortsignal auszusenden. Die Transponder 4 und 8 sind somit als Hybridtags ausgebildet. Die Transponder 4 und 8 weisen jeweils keinen eigenen Energiespeicher auf und sind somit passiv ausgebildet. Nur bei einem Empfang von vorbestimmten Signalen senden die Transponder 4 und 8 entsprechende Antwortsignale. Die Antwortsignale enthalten jeweils Informationen, welche das Antwortsignal eindeutig dem entsprechenden Transponder 4 oder 8 und damit eindeutig dem entsprechenden Medizinprodukt 2 oder 6 zuordnen.

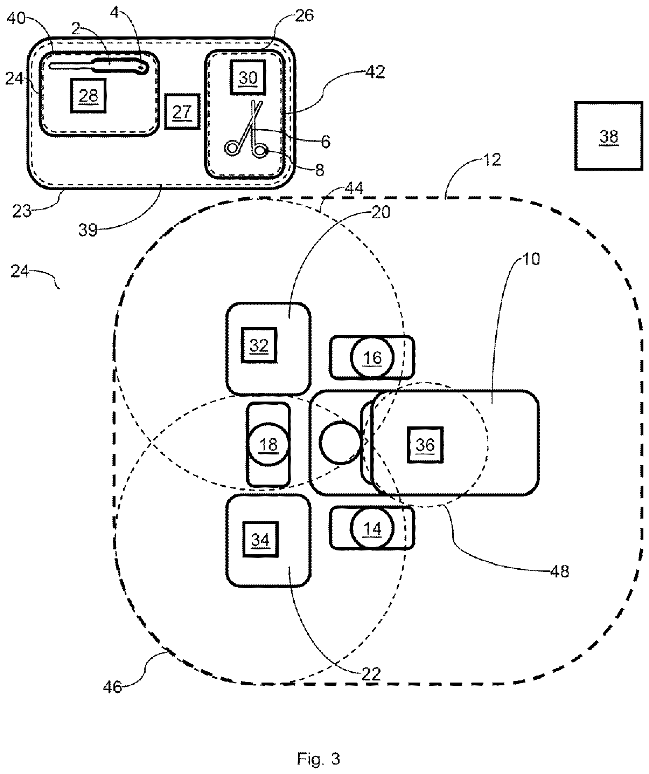

Fig. 3 zeigt eine schematische Draufsicht auf einen Operationssaal, der mit einem offenbarungsgemäßen System ausgestattet ist. Der Operationssaal weist einen Operationstisch 10 auf, um den sich ein Sterilbereich 12 erstreckt. Innerhalb des Sterilbereichs 12 stehen eine Chirurgin 14, eine Assistenz 16 und eine Instrumentierschwester 18 rund um den Operationstisch 10. Beidseits der an einer Stirnseite des Operationstisch 10 stehenden Instrumentierschwester 18 sind zwei Beistelltische 20 und 22 angeordnet. Abseits des Sterilbereichs 12 ist in dem Operationssaal ein Lagertisch 23 angeordnet. Auf dem Lagertisch 23 ist das Mikromotorinstrument 2 innerhalb eines Sterilgutbehälters 24 gelagert und ist der Nadelhalter 6 innerhalb eines Sterilgutbehälters 26 gelagert.

An dem Lagertisch 23 ist eine Überwachungsvorrichtung 27 vorgesehen. An bzw. in dem Sterilgutbehälter 24 ist eine Überwachungsvorrichtung 28 vorgesehen. An bzw. in dem Sterilgutbehälter 26 ist eine Überwachungsvorrichtung 30 vorgesehen. An dem Instrumententisch 20 ist eine Überwachungsvorrichtung 32 vorgesehen. An dem Instrumententisch 22 ist eine Überwachungsvorrichtung 34 vorgesehen. An dem Operationstisch 10 ist eine Überwachungsvorrichtung 36 vorgesehen. Die Überwachungsvorrichtungen 27 bis 36 sind jeweils mit einer Datenverarbeitungsvorrichtung 38 sowie miteinander nicht-kabelgebunden über ein WLAN-Standard-Netzwerk, wie zum Beispiel über ein Bluetooth^{®}-Netzwerk oder ein BLE-Netzwerk, verbunden.

Um jede der Überwachungsvorrichtungen 27 bis 36 erstreckt sich ein Erkennungsbereich 39, 40, 42, 44, 46 und 48.

Die Überwachungsvorrichtung 27 ist derart ausgebildet, dass Grenzen des Erkennungsbereichs 39 der Überwachungsvorrichtung 27 zumindest im Wesentlichen einem Umriss des Lagertisches 23 entsprechen. Die Überwachungsvorrichtung 27 ist ausgebildet, den Transponder 4 bzw. 8 mittels eines Nahfeldkommunikationssignal zu erkennen.

Der Erkennungsbereich 40 der Überwachungsvorrichtung 28 und der Erkennungsbereich 42 der Überwachungsvorrichtung 30 sind jeweils von dem entsprechenden Sterilgutbehälter 24 bzw. 26, in welchem das Mikromotorinstrument 2 bzw. der Nadelhalter 6 gelagert ist, begrenzt. Die Überwachungsvorrichtungen 28 und 30 sind ausgebildet, den Transponder 4 bzw. 8 mittels eines Nahfeldkommunikationssignals zu erkennen.

Die Überwachungsvorrichtungen 32 und 34 sind ausgebildet, den Transponder4 bzw. 8 mittels eines RFID-Signals zu erkennen. Dementsprechend sind der Erkennungsbereich 44 der Überwachungsvorrichtung 32 und der Erkennungsbereich 46 der Überwachungsvorrichtung 34 größer als die Erkennungsbereiche 40 und 42.

Die Überwachungsvorrichtung 36 ist ebenfalls ausgebildet, den Transponder4 bzw. 8 mittels eines RFID-Signals zu erkennen. Die Intensität des RFID-Signal der Überwachungsvorrichtung 36 ist allerdings geringer als die Intensität der RFID Signale der Überwachungsvorrichtungen 32 und 34. Dementsprechend ist der Erkennungsbereich 48 der Überwachungsvorrichtung 36 kleiner als die Erkennungsbereiche 44 und 46 der Überwachungsvorrichtungen 32 und 34.

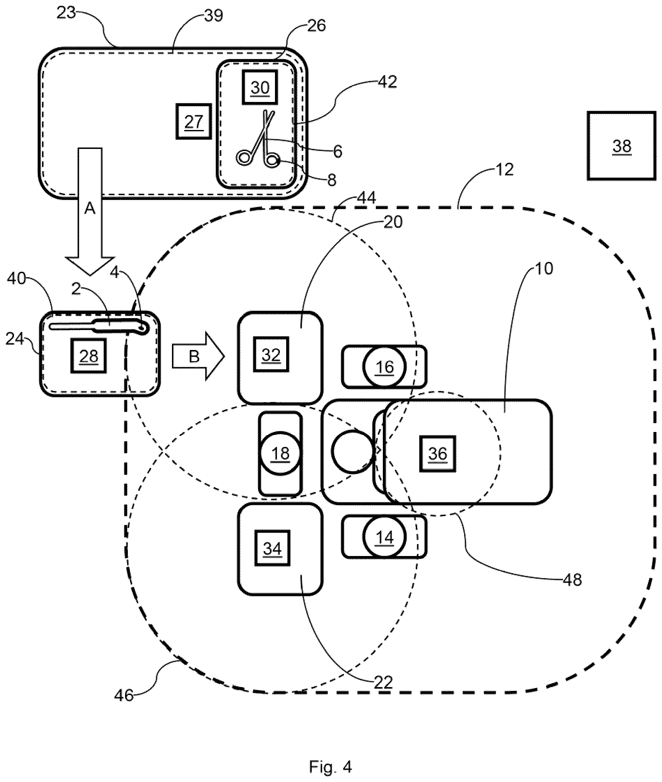

Fig. 4 zeigt eine schematische Ansicht des Systems gemäß Fig. 3 in einem Zustand, in welchem der Sterilgutbehälter 24 von dem Lagertisch 23 entfernt wurde (siehe Pfeil A) und sich der Sterilgutbehälter 24 außerhalb eines durch den Lagertisch 23 vorgegebenen Lagerbereichs und teilweise innerhalb des Sterilbereichs 12 befindet. In dem in Fig. 4 gezeigten Zustand befindet sich der Transponder 4 sowohl in dem Erkennungsbereich 40 des Sterilgutbehälters 24 als auch in dem Erkennungsbereich 44 der Überwachungsvorrichtung 32 des Beistelltischs 20. Dadurch, dass sowohl die Überwachungsvorrichtung 28 als auch die Überwachungsvorrichtung 32 den Transponder 4 erkennt, kann das System bzw. die Datenverarbeitungsvorrichtung 38 erkennen, dass das Mikromotorinstrument 2 noch in dem Sterilgutbehälter 24 ist und dass sich der Sterilgutbehälter 24 zumindest teilweise im Erkennungsbereich 44 und somit zumindest teilweise in dem Sterilbereich 12 befindet. Bei einem Bewegen des Sterilgutbehälters 24 vom Rand des Erkennungsbereiches 44 in Richtung des Beistelltischs 20 (siehe Pfeil B) registriert das System bzw. die Datenverarbeitungsvorrichtung 38 keine Zustandsänderung, da bei einer solchen Bewegung weiterhin nur die Überwachungsvorrichtungen 28 und 32 den Transponder erkennen.

In dem in Fig. 4 gezeigten Zustand befindet sich das Mikromotorinstrument 2 und der Sterilgutbehälter 24 nicht mehr auf dem Lagertisch 23. Der Transponder 4 befindet sich somit nicht mehr im Erkennungsbereich 39, sondern in dem Erkennungsbereich 44. Der Zeitpunkt, ab welchem der Transponder 4 nicht mehr von der Überwachungsvorrichtung 27 erkannt wird und sich der Transponder 4 also nicht in dem Erkennungsbereich 39 befindet, wird von der Überwachungsvorrichtung 27 an die Datenverarbeitungsvorrichtung 38 gemeldet. Ebenso wird der Zeitpunkt, ab welchem der Transponder 4 von der Überwachungsvorrichtung 32 erkannt wird und sich der Transponder also in dem Erkennungsbereich 44 befindet, von der Überwachungsvorrichtung 32 an die Datenverarbeitungsvorrichtung 38 gemeldet.

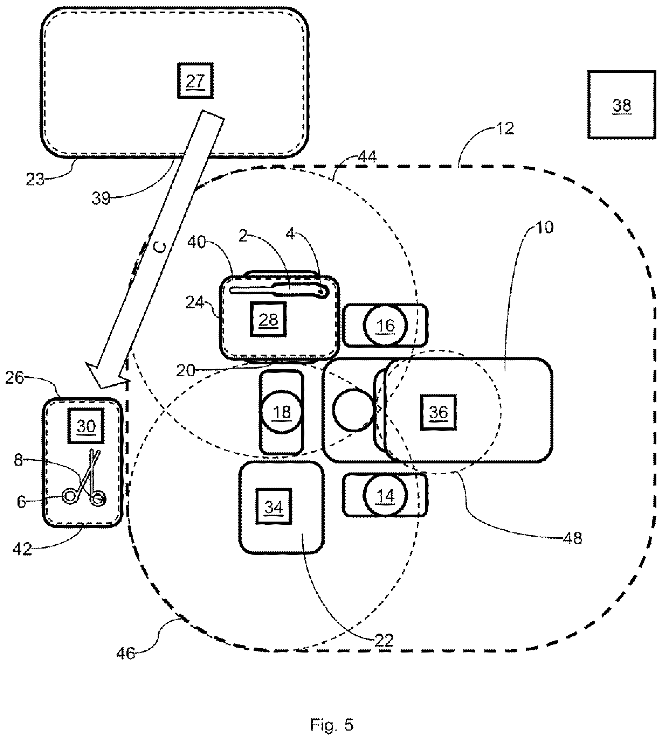

In dem in Fig. 5 gezeigten Zustand befindet sich der Sterilgutbehälter 24 mit dem Mikromotorinstrument 2 auf dem Beistelltisch 20. Gemäß der gezeigten Ausführungsform, kann die Datenverarbeitungsvorrichtung 38 den in Fig. 5 gezeigten Zustand des Mikromotorinstruments 2 bzw. Sterilgutbehälters 24 nicht von dem in Fig. 4 gezeigten Zustand unterscheiden. Für eine verbesserte Überwachung kann die Überwachungsvorrichtung 32 derart ausgebildet sein, dass sie erkennen kann, ob sich ein Transponder bzw. der Transponder 4 in einem sich um die Überwachungsvorrichtung 32 erstreckenden Naherkennungsbereich befindet, welcher kleiner als der Erkennungsbereich 44 ist und sich insbesondere im Wesentlichen entsprechend dem Grundriss des Beistelltischs 20 erstreckt.

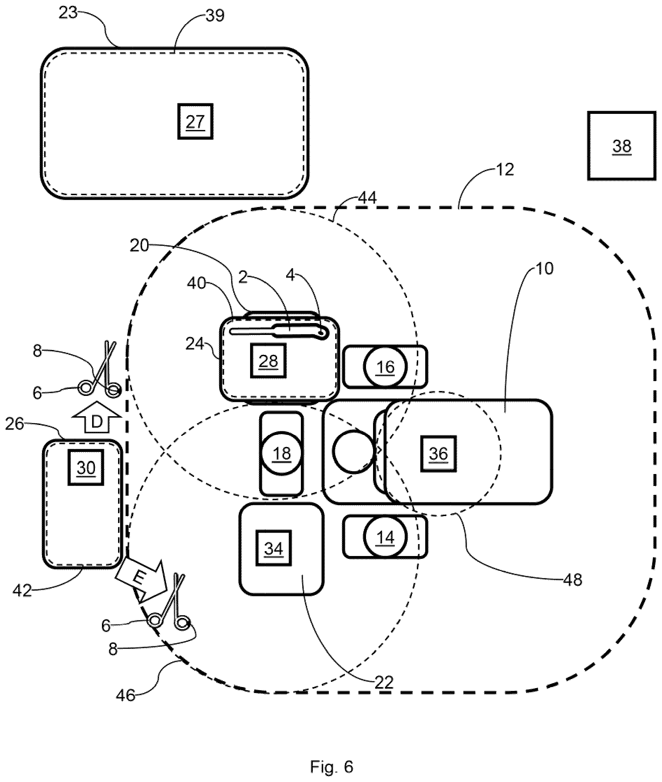

In dem in Fig. 5 gezeigten Zustand ist der Sterilgutbehälter 26 von dem Lagertisch 23 entfernt (siehe Pfeil C) und befindet sich der Sterilgutbehälter 26 mitsamt dem Nadelhalter 6 und dem Transponder 8 außerhalb des durch den Lagertisch 23 vorgegebenen Lagerbereichs und außerhalb des Sterilbereichs 12. Wird in diesem Zustand der Nadelhalter 6 aus dem Sterilgutbehälter 26 genommen (siehe Pfeile D und Ein Fig. 6), meldet die Überwachungsvorrichtung 30 des Sterilgutbehälters 26 der Datenverarbeitungsvorrichtung 38, dass sie den Transponder 8 nicht mehr erkennt und die Datenverarbeitungsvorrichtung 38 registriert den entsprechenden Zeitpunkt. Durch Abgleich mit von den übrigen Überwachungsvorrichtungen 27, 28, 34 und 36 empfangenen Daten erkennt die Datenverarbeitungsvorrichtung 38, dass der Nadelhalter 6 bei bzw. unmittelbar nach seiner Entnahme aus dem außerhalb des Lagerbereichs und außerhalb des Sterilbereichs angeordneten Sterilgutbehälters von keiner der Überwachungsvorrichtungen 27 bis 36 erkannt wird. Die Datenverarbeitungsvorrichtung 38 gemäß der Ausführungsform ist ausgebildet, bei einem solchen Verschwinden eines bereits erkannten Transponders 8 aus den Erkennungsbereichen 39 bis 48 einen Alarm und/oder eine Warnung auszugeben. Dabei ist es unerheblich, ob der Transponder 8 kurze Zeit nach dem Verschwinden aus dem Erkennungsbereich 42 von einer anderen Überwachungsvorrichtung beispielsweise von der Überwachungsvorrichtung 34 des Beistelltischs 22 erkannt wird (siehe Pfeil E in Fig. 6).

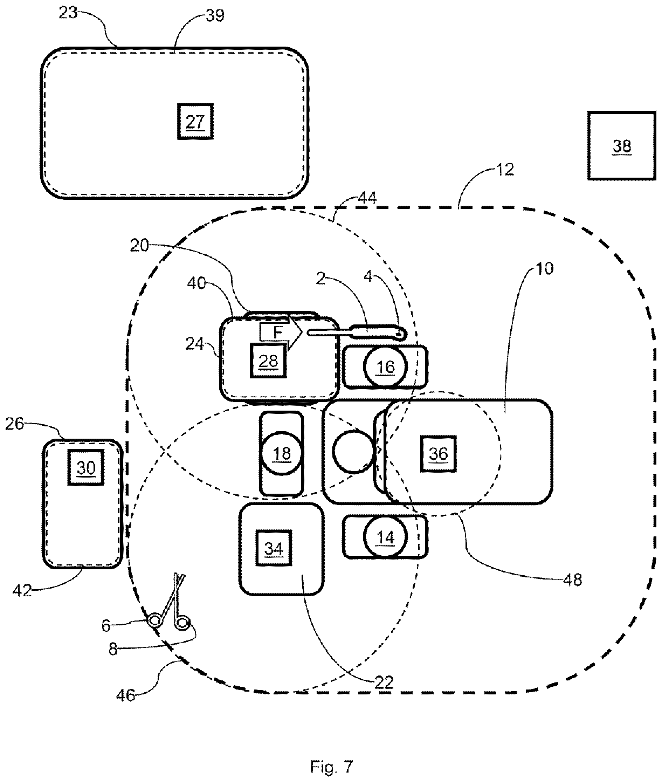

In dem in Fig. 7 gezeigten Zustand ist das Mikromotorinstrument 2 dem auf dem Beistelltisch 20 ruhenden Sterilgutbehälter 24 entnommen (siehe Pfeil F) und befindet sich das Mikromotorinstrument 2 bzw. der Transponder 4 ausschließlich in dem Erkennungsbereich 44 der Überwachungsvorrichtung 32 des Beistelltischs 20. Der Erkennungsbereich 44 der Überwachungsvorrichtung 32 des Beistelltischs 20 ist derart ausgebildet, dass er sich ausschließlich in dem Sterilbereich 12 erstreckt, so dass eine Entnahme des Mikromotorinstruments 2 innerhalb des Erkennungsbereichs 44 zu keiner Alarm- und/oder Warnmeldung durch die Datenverarbeitungsvorrichtung 38 führt.

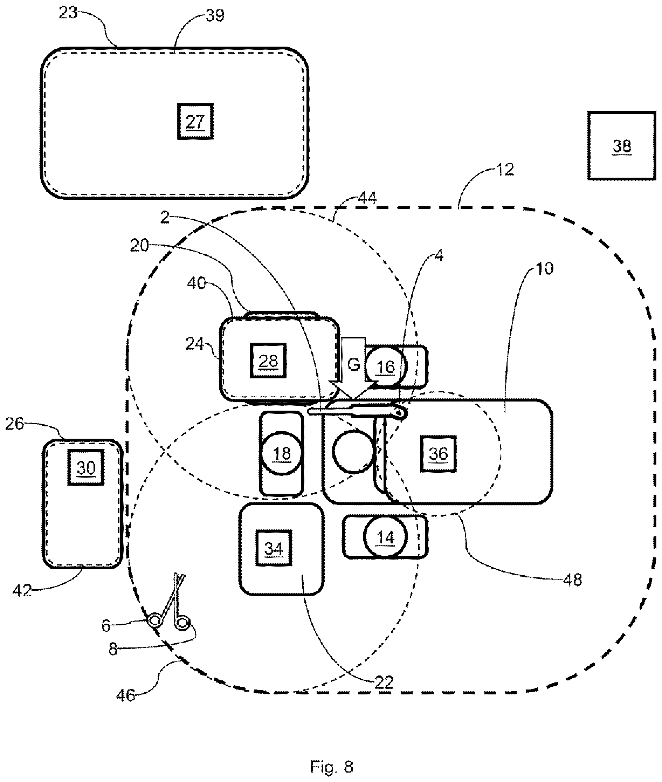

In dem in Fig. 8 gezeigten Zustand wurde das Mikromotorinstrument 2 in Richtung des Operationstischs 10 bewegt (siehe Pfeil G) und befindet sich der Transponder 4 des Mikromotorinstruments 2 in einem Überlappungsbereich des Erkennungsbereichs 44 der Überwachungsvorrichtung 32 des Beistelltischs 20 und des Erkennungsbereichs 48 der Überwachungsvorrichtung 36 an dem Operationstisch 10. Sobald der Transponder 4 beim Bewegen des Mikromotorinstruments 2 in Richtung des Operationstischs 10 den Erkennungsbereich 48 erreicht und somit von der Überwachungsvorrichtung 36 erkannt wird, meldet die Überwachungsvorrichtung 36 den entsprechenden Zeitpunkt an die Datenverarbeitungsvorrichtung 38.

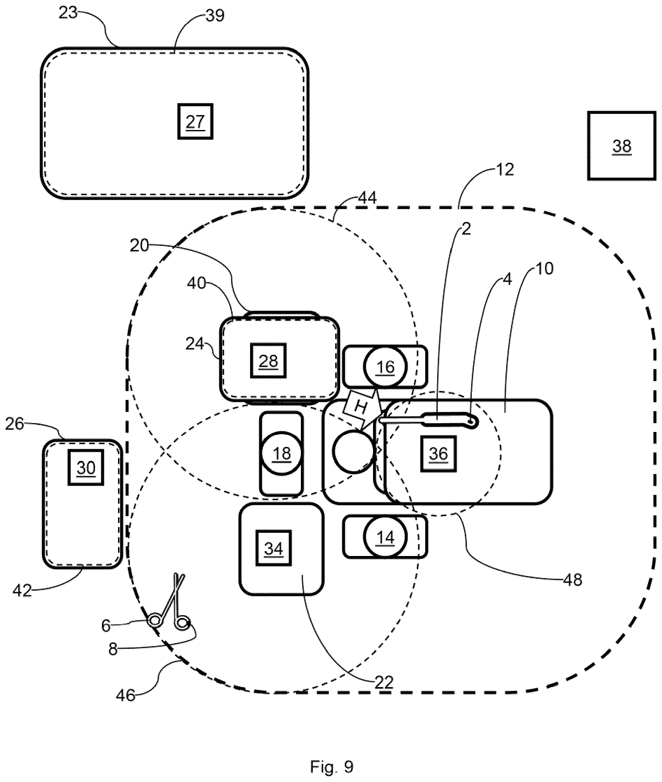

In dem in Fig. 9 gezeigten Zustand wurde das Mikromotorinstrument 2 weiter in Richtung des Operationstischs 10 bewegt (siehe Pfeil H) und befindet sich der Transponder 4 des Mikromotorinstruments 2 nunmehr ausschließlich im Erkennungsbereich 48 der Überwachungsvorrichtung 36 des Operationstischs 10. Der Zeitpunkt, ab welchem der Transponder 4 nicht mehr von der Überwachungsvorrichtung 32 erkannt wird und sich der Transponder 4 also nicht mehr in dem Erkennungsbereich 44 befindet, wird von der Überwachungsvorrichtung 32 an die Datenverarbeitungsvorrichtung 38 gemeldet.

Anhand der von den Überwachungsvorrichtungen 27, 28, 32 und 36 an die Datenverarbeitungsvorrichtung 38 gemeldeten Zeitpunkte der Erkennungen bzw. der Nicht-mehr-Erkennungen des Transponders 4 in den Erkennungsbereichen 39, 40, 44 und 48 kann die Datenverarbeitungsvorrichtung 38 die Zeitdauern berechnen, während derer sich der Transponder 4 in dem jeweiligen Erkennungsbereich 39, 40, 44 und 48 befindet. Wird das Mikromotorinstrument 2 in umgekehrter Reihenfolge wieder von dem Operationstisch 10 zu dem Lagertisch 23 bewegt, werden die Zeitpunkte der Erkennungen des Transponders durch die Überwachungsvorrichtungen entsprechend an die Datenverarbeitungsvorrichtung 38 gemeldet und die Datenverarbeitungsvorrichtung 38 berechnet wiederum die Zeitdauern, während derer sich der Transponder 4 in dem jeweiligen Erkennungsbereich 39, 40, 44 und 48 befindet.

Durch das in den Fig. 3 bis Fig. 9 gezeigte offenbarungsgemäße System ist es möglich, die Gesamtaufenthaltszeitdauern des Mikromotorinstruments 2 in den einzelnen Erkennungsbereichen 39, 40, 44 und 48 zu berechnen und somit eine kohärente Überwachung des Mikromotorinstruments 2 zu erreichen.

Eine Überwachung des Nadelhalters 6 kann entsprechend der Überwachung des Mikromotorinstruments 2 ausgeführt werden.

In der Datenverarbeitungsvorrichtung 38 kann zumindest ein vorbestimmter Ablauf einer Operation abgespeichert sein, aus welchem eine Reihenfolge zumindest zweier für die Operation zu verwendender Instrumente hervorgeht. Muss zum Beispiel bei einer Operation zunächst ein Mikromotorinstrument 4 und dann ein Nadelhalter 8 verwendet werden, kann die Datenverarbeitungsvorrichtung 38 während der Operation entsprechende Assistenzmeldungen und/oder bei einem Abweichen von dem vorbestimmten Ablauf, also beispielsweise dann, wenn der Nadelhalter 8 vor dem Mikromotorinstrument 4 von der Überwachungsvorrichtung 36 des Operationstisches 10 erkannt wird, eine Alarm- oder Warnmeldung ausgeben. Auch kann die Datenverarbeitungsvorrichtung 38 derart ausgebildet sein, dass sie, wenn zum Ende einer Operation die Instrumente 2 und 6, insbesondere die wiederverwendbaren Instrumente, nicht von den Überwachungsvorrichtungen 28 und 30 in den entsprechenden Sterilgutbehältern 24 und 26 erkannt werden und/oder nicht von der Überwachungsvorrichtung 27 an dem Lagertisch 23 erkannt werden, eine Alarm- oder Warnmeldung ausgibt.

Durch Verwendung eines Transponders in Form eines Hybridtags ist es auf einfache Weise möglich, unterschiedlich große Erkennungsbereiche bereitzustellen und somit die Form eines überwachten Bereichs flexibel zu gestalten. Werden bei einer Berechnung von Zeitdauern nur Zustände berücksichtigt, bei welchen nur eine einzelne Überwachungsvorrichtung einen Transponder erkennt, kann mittels Überlappungen mehrerer Erkennungsbereiche verschiedener Überwachungsvorrichtungen ein Bereich mit einer komplexen Form überwacht werden.

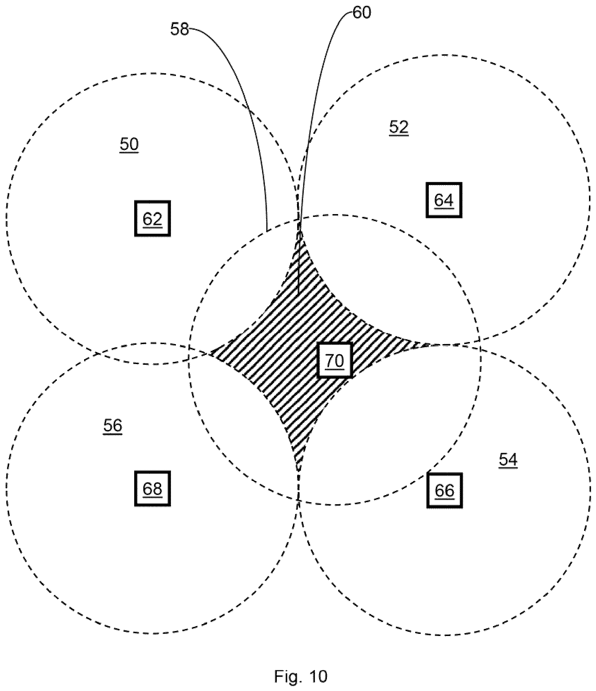

Fig. 10 zeigt eine schematische Ansicht eines durch Überlappung mehrerer Erkennungsbereiche 50, 52, 54, 56 und 58 gebildeten Überwachungsbereichs 60. Dazu werden beispielsweise vier Überwachungsvorrichtungen 62, 64, 66 und 68 um eine zentrale Überwachungsvorrichtung 70 derart angeordnet, dass Überlappungen der Erkennungsbereiche 50, 52, 54 und 56 der außen angeordneten Überwachungsvorrichtungen 62, 64, 66 und 68 mit dem Erkennungsbereich 58 der zentral angeordneten Überwachungsvorrichtung 70 einen mit keinem anderen Erkennungsbereich überlappenden Teil des Erkennungsbereich 58 der zentral angeordneten Überwachungsvorrichtung 70 begrenzen. Der mit keinem anderen Erkennungsbereich überlappende Teil des Erkennungsbereich 58 der zentral angeordneten Überwachungsvorrichtung 70 stellt den Überwachungsbereich 60 dar, in welchem Zeitdauern eines Aufenthalts eines Transponders offenbarungsgemäß berechnet werden können.

### Bezugszeichenliste

- 2: Mikromotorinstrument
- 4: Transponder
- 6: Nadelhalter
- 8: Transponder
- 10: Operationstisch
- 12: Sterilbereich
- 14: Chirurgin
- 16: Assistenz
- 18: Instrumentierschwester
- 20, 22: Beistelltisch
- 23: Lagertisch
- 24, 26: Sterilgutbehälter
- 27 - 36: Überwachungsvorrichtung
- 38: Datenverarbeitungsvorrichtung
- 39 - 48: Erkennungsbereich
- 50 - 58: Erkennungsbereich
- 60: Überwachungsbereich
- 62 - 70: Überwachungsvorrichtung

## Patentansprüche

1. System zur Überwachung zumindest eines Medizinprodukts (2, 6) mit
zumindest zwei miteinander in einem Überwachungsnetzwerk datenleitend verbundenen oder verbindbaren Überwachungsvorrichtungen (27 - 36), die jeweils ein Erkennungsgerät aufweisen, das ausgebildet ist, zu erkennen, ob sich in einem sich um die jeweilige Überwachungsvorrichtung (27 - 36) erstreckenden Erkennungsbereich (39 - 48) ein Transponder (4, 8) des Medizinprodukts (2, 6) befindet, wobei die zumindest zwei Überwachungsvorrichtungen (27 - 36) derart angeordnet sind, dass sich die Erkennungsbereiche (39 - 48) der zumindest zwei Überwachungsvorrichtungen (27 - 36) überlappen,
einer mit den zumindest zwei Überwachungsvorrichtungen (27 - 36) über das Überwachungsnetzwerk datenleitend verbundenen oder verbindbaren Datenverarbeitungsvorrichtung (38), die ausgebildet ist, Zeitpunkte von Erkennungen des Transponders (4, 8) durch die Überwachungsvorrichtungen (27 - 36) zu speichern, und
einem Speicher,
**dadurch gekennzeichnet, dass**
die Datenverarbeitungsvorrichtung (38) ausgebildet ist,
Zeitdauern, in welchen sich der Transponder (4, 8) ausschließlich in einem der Erkennungsbereiche (39 - 48) der zumindest zwei Überwachungsvorrichtungen (27 - 36) befindet, zu berechnen, die berechneten Zeitdauern mit entsprechenden im Speicher gespeicherten Soll-Zeitdauern zu vergleichen und bei einem Überschreiten von Soll-Zeitdauern entsprechende Alarmsignale auszugeben und/oder
Reihenfolgen, in welchen die zumindest zwei Überwachungsvorrichtungen (27 - 36) den Transponder (4, 8) in den Erkennungsbereichen (39 - 48) erkennen, zu registrieren, die registrierten Reihenfolgen mit entsprechenden im Speicher gespeicherten Soll-Reihenfolgen zu vergleichen und bei einem Abweichen von Soll-Reihenfolgen entsprechende Alarmsignale auszugeben.

2. System nach Anspruch 1, **dadurch gekennzeichnet, dass** das Medizinprodukt (2, 6) widerverwendbar und/oder ein chirurgisches Instrument ist.

3. System nach Anspruch 1 oder 2, **dadurch gekennzeichnet, dass** die zumindest zwei Überwachungsvorrichtungen (27 - 36) derart ausgebildet sind, dass die zugehörigen Erkennungsbereiche (39 - 48) unterschiedlich groß sind.

4. System nach einem der Ansprüche 1 bis 3, **dadurch gekennzeichnet, dass** zumindest eines der zumindest zwei Erkennungsgeräte ausgebildet ist, erkennen zu können, ob sich der Transponder des Medizinprodukts in einem sich um die jeweilige Überwachungsvorrichtung erstreckenden und im Vergleich zum entsprechenden Erkennungsbereich kleineren Naherkennungsbereich befindet und/oder ausgebildet ist, erkennen zu können, ob sich der Transponder des Medizinprodukts in einem sich um die jeweilige Überwachungsvorrichtung erstreckenden und im Vergleich zum entsprechenden Erkennungsbereich größeren Fernerkennungsbereich befindet.

5. System nach Anspruch 4, **dadurch gekennzeichnet, dass** die Datenverarbeitungsvorrichtung ausgebildet ist, Zeitdauern, in welchen sich der Transponder in dem zumindest einen Naherkennungsbereich und/oder in dem zumindest einen Fernerkennungsbereich befindet, berechnen zu können.

6. System nach Anspruch 4 oder 5, **dadurch gekennzeichnet, dass** die Datenverarbeitungsvorrichtung ausgebildet ist, Zeitdauern, in welchen sich der Transponder nicht in dem zumindest einen Naherkennungsbereich und/oder nicht in dem zumindest einen Fernerkennungsbereich befindet, berechnen zu können.

7. System nach einem der Ansprüche 1 bis 6, **dadurch gekennzeichnet, dass**
zumindest eines der Erkennungsgeräte ausgebildet ist, zur Erkennung des Transponders (4, 8) elektromagnetische Wellen eines ersten Frequenzbandes aussenden und von dem Transponder (4, 8) empfangen zu können, und
zumindest eines der Erkennungsgeräte ausgebildet ist, zur Erkennung des Transponders (4, 8) elektromagnetische Wellen eines zweiten Frequenzbandes aussenden und von dem Transponder (4, 8) empfangen zu können.

8. System nach einem der Ansprüche 1 bis 7, **dadurch gekennzeichnet, dass**
zumindest eines der Erkennungsgeräte ausgebildet ist, zur Erkennung des Transponders (4, 8) elektromagnetische Wellen gemäß einem ersten Übertragungsstandard auszusenden und von dem Transponder (4, 8) zu empfangen, und
zumindest eines der Erkennungsgeräte ausgebildet ist, zur Erkennung des Transponders (4, 8) elektromagnetische Wellen gemäß einem zweiten Übertragungsstandard auszusenden und von dem Transponder (4, 8) zu empfangen

9. System nach einem der Ansprüche 1 bis 8, **dadurch gekennzeichnet, dass** das Überwachungsnetzwerk ausgebildet ist, gemäß einem dritten Übertragungsstandard betrieben zu werden.

10. System nach einem der Ansprüche 1 bis 9, **dadurch gekennzeichnet, dass** das Überwachungsnetzwerk teilweise oder vollständig vermascht ist.

11. System nach einem der Ansprüche 1 bis 10, **dadurch gekennzeichnet, dass** zumindest eine Überwachungsvorrichtung (28; 30) in einem Sterilgutbehälter (24; 26) angeordnet ist.

12. System nach einem der Ansprüche 1 bis 11, **dadurch gekennzeichnet, dass**
zumindest eine Überwachungsvorrichtung (27) in einem Lagerungsbereich einer Operationsumgebung angeordnet ist,
zumindest eine Überwachungsvorrichtung (32, 34) in der Operationsumgebung in einem Sterilbereich (12) an einem Beistelltisch (20, 22) in der Umgebung eines Operationstisches (10) angeordnet ist und
zumindest eine Überwachungsvorrichtung (36) in der Operationsumgebung in dem Sterilbereich (12) an dem Operationstisch (10) angeordnet ist.

13. System nach einem der Ansprüche 1 bis 12, **gekennzeichnet durch** ein Positionierungssystem, das ausgebildet ist, relative Positionen der zumindest zwei Überwachungsvorrichtungen (27 - 36) detektieren und/oder registrieren zu können.

14. Verfahren zur Überwachung zumindest eines Medizinprodukts (2, 6) in einer Operationsumgebung, in welcher zumindest zwei miteinander in einem Überwachungsnetzwerk datenleitend verbundenen oder verbindbaren Überwachungsvorrichtungen (27 - 36), derart angeordnet oder anordenbar sind, dass sich um die jeweilige Überwachungsvorrichtung (27 - 36) erstreckende Erkennungsbereiche (39 - 48) der zumindest zwei Überwachungsvorrichtungen (27 - 36) überlappen, wobei das Medizinprodukt (2,6) insbesondere wiederverwendbar und/oder ein chirurgisches Instrument ist, mit den Schritten
- Erkennen, ob sich in einem der Erkennungsbereiche (39 - 48) ein Transponder (4, 8) des Medizinprodukts (2, 6) befindet, und
- Speichern von Zeitpunkten von Erkennungen des Transponders (4, 8),
**gekennzeichnet durch**
- Berechnen von Zeitdauern, in welchen sich der Transponder (4, 8) ausschließlich in einem der Erkennungsbereiche (39 - 48) der zumindest zwei Überwachungsvorrichtungen (27 - 36) befindet, Vergleichen der berechneten Zeitdauern mit entsprechenden gespeicherten Soll-Zeitdauern und Ausgeben entsprechender Alarmsignale bei einem Überschreiten von Soll-Zeitdauern und/oder
- Registrieren von Reihenfolgen, in welchen die zumindest zwei Überwachungsvorrichtungen (27 - 36) den Transponder (4, 8) in den Erkennungsbereichen (39 - 48) erkennen, Vergleichen der registrierten Reihenfolgen mit entsprechenden gespeicherten Soll-Reihenfolgen und Ausgeben entsprechender Alarmsignale bei einem Abweichen von Soll-Reihenfolgen.

## Claims

1. A system for monitoring at least one medical product (2, 6) with
at least two monitoring devices (27 - 36) which are connected or can be connected to each other in a monitoring network in a data-conducting manner and which each have a detection device which is configured to detect whether a transponder (4, 8) of the medical product (2, 6) is located in a detection region (39 - 48) extending around the respective monitoring device (27 - 36), wherein the at least two monitoring devices (27 - 36) are arranged in such a way that the detection regions (39 - 48) of the at least two monitoring devices (27 - 36) overlap,
a data processing device (38) which is connected or connectable in a data-conductive manner to the at least two monitoring devices (27 - 36) via the monitoring network and which is configured to store times of detections of the transponder (4, 8) by the monitoring devices (27 - 36), and
a storage device,
**characterized in that**
the data processing device (38) is configured
to calculate periods in which the transponder (4, 8) is located exclusively in one of the detection regions (39 - 48) of the at least two monitoring devices (27 - 36), to compare the calculated periods with corresponding target periods stored in the storage device and to output corresponding alarm signals if target periods are exceeded, and/or
to register sequences in which the at least two monitoring devices (27 - 36) detect the transponder (4, 8) in the detection regions (39 - 48), to compare the registered sequences with corresponding target sequences stored in the storage device and to output corresponding alarm signals in the event of a deviation from target sequences.

2. The system according to claim 1, **characterized in that** the medical product (2, 6) is reusable and/or a surgical instrument.

3. The system according to claim 1 or 2, **characterized in that** the at least two monitoring devices (27 - 36) are configured such that the associated detection regions (39 - 48) are of different sizes.

4. The system according to one of claims 1 to 3, **characterized in that** at least one of the at least two detection devices is configured to be able to detect whether the transponder of the medical product is located in a proximity detection region extending around the respective monitoring device and being smaller compared to the corresponding detection region and/or is configured to be able to detect whether the transponder of the medical product is located in a remote detection region extending around the respective monitoring device and being larger compared to the corresponding detection region.

5. The system according to claim 4, **characterized in that** the data processing device is configured to be able to calculate periods in which the transponder is located in the at least one proximity detection region and/or in the at least one remote detection region.

6. The system according to claim 4 or 5, **characterized in that** the data processing device is configured to be able to calculate periods in which the transponder is not in the at least one proximity detection region and/or not in the at least one remote detection region.

7. The system according to one of claims 1 to 6, **characterized in that**
at least one of the detection devices is configured to emit and receive electromagnetic waves of a first frequency band to and from the transponder (4, 8) for detecting the transponder (4, 8), and
at least one of the detection devices is configured to emit electromagnetic waves of a second frequency band for detecting the transponder (4, 8) and to receive them from the transponder (4, 8).

8. The system according to one of claims 1 to 7, **characterized in that**
at least one of the detection devices is configured to transmit to and receive electromagnetic waves from the transponder (4, 8) according to a first transmission standard for detecting the transponder (4, 8), and
at least one of the detection devices is configured to transmit and receive electromagnetic waves to and from the transponder (4, 8) according to a second transmission standard in order to detect the transponder (4, 8)

9. The system according to one of claims 1 to 8, **characterized in that** the monitoring network is configured to be operated according to a third transmission standard.

10. The system according to one of claims 1 to 9, **characterized in that** the monitoring network is partially or completely meshed.

11. The system according to one of claims 1 to 10, **characterized in that** at least one monitoring device (28; 30) is arranged in a sterile container (24; 26).

12. The system according to one of claims 1 to 11, **characterized in that**
at least one monitoring device (27) is arranged in a storage area of a surgery environment,
at least one monitoring device (32, 34) in the surgery environment is arranged in a sterile area (12) on a side table (20, 22) in the vicinity of an operating table (10), and
at least one monitoring device (36) is arranged in the surgery environment in the sterile area (12) on the operating table (10).

13. The system according to one of claims 1 to 12, **characterized by a** positioning system configured to detect and/or to register relative positions of the at least two monitoring devices (27 - 36).

14. A method for monitoring at least one medical product (2, 6) in a surgical environment, in which at least two monitoring devices (27 - 36), which are connected or can be connected to each other in a monitoring network in a data-conducting manner, are arranged or can be arranged in such a way that detection regions (39 - 48) of the at least two monitoring devices (27 - 36) extending around the respective monitoring device (27 - 36) overlap, wherein the medical product (2, 6) is in particular reusable and/or is a surgical instrument, comrpising the following steps
- detecting whether a transponder (4, 8) of the medical product (2, 6) is located in one of the detection regions (39 - 48), and
- storing times of detections of the transponder (4, 8),
**characterized by**
- calculating periods in which the transponder (4, 8) is exclusively located in one of the detection regions (39 - 48) of the at least two monitoring devices (27 - 36), comparing the calculated periods with corresponding stored target periods and outputting corresponding alarm signals when target periods are exceeded and/or
- Registering sequences in which the at least two monitoring devices (27 - 36) detect the transponder (4, 8) in the detection regions (39 - 48), comparing the registered sequences with corresponding stored target sequences and outputting corresponding alarm signals in the event of a deviation from target sequences.

## Revendications

1. Système de surveillance d'au moins un produit médical (2, 6) avec
au moins deux dispositifs de surveillance (27-36) reliés ou pouvant être reliés l'un à l'autre par conduction de données dans un réseau de surveillance, lesquels présentent chacun un appareil de détection qui est réalisé de sorte à détecter si un transpondeur (4, 8) du produit médical (2, 6) se trouve dans une zone de détection (39-48) s'étendant autour du dispositif de surveillance (27-36) respectif, dans lequel les au moins deux dispositifs de surveillance (27-36) sont agencés de sorte que les zones de détection (39-48) des au moins deux dispositifs de surveillance (27-36) se chevauchent,
un dispositif de traitement de données (38) relié ou pouvant être relié par conduction de données aux au moins deux dispositifs de surveillance (27-36) par l'intermédiaire du réseau de surveillance, lequel est réalisé de sorte à mémoriser des moments de détections du transpondeur (4, 8) par les dispositifs de surveillance (27-36), et
une mémoire,
**caractérisé en ce que**
le dispositif de traitement de données (38) est réalisé pour
calculer des durées pendant lesquelles le transpondeur (4, 8) se trouve exclusivement dans l'une des zones de détection (39-48) des au moins deux dispositifs de surveillance (27-36), pour comparer les durées calculées avec des durées de consigne correspondantes mémorisées dans la mémoire et pour émettre des signaux d'alarme correspondants en cas de dépassement de durées de consigne et/ou
enregistrer des séquences dans lesquelles les au moins deux dispositifs de surveillance (27-36) détectent le transpondeur (4, 8) dans les zones de détection (39-48), pour comparer les séquences enregistrées avec des séquences de consigne correspondantes stockées dans la mémoire et pour émettre des signaux d'alarme correspondants en cas d'écart par rapport aux séquences de consigne.

2. Système selon la revendication 1, **caractérisé en ce que** le produit médical (2, 6) est réutilisable et/ou est un instrument chirurgical.

3. Système selon la revendication 1 ou 2, **caractérisé en ce que** les au moins deux dispositifs de surveillance (27-36) sont réalisés de sorte que les zones de détection associées (39-48) sont de tailles différentes.

4. Système selon l'une quelconque des revendications 1 à 3, **caractérisé en ce qu'**au moins un des au moins deux appareils de détection est réalisé de sorte à pouvoir détecter si le transpondeur du produit médical se trouve dans une zone de détection de proximité s'étendant autour du dispositif de surveillance respectif et est plus petite par rapport à la zone de détection correspondante et/ou est réalisé de sorte à pouvoir détecter si le transpondeur du produit médical se trouve dans une zone de détection à distance s'étendant autour du dispositif de surveillance respectif et est plus grande par rapport à la zone de détection correspondante.

5. Système selon la revendication 4, **caractérisé en ce que** le dispositif de traitement de données est réalisé de sorte à pouvoir calculer des durées pendant lesquelles le transpondeur se trouve dans l'au moins une zone de détection de proximité et/ou dans l'au moins une zone de détection de distance.

6. Système selon la revendication 4 ou 5, **caractérisé en ce que** le dispositif de traitement de données est réalisé de sorte à pouvoir calculer des durées pendant lesquelles le transpondeur ne se trouve pas dans ladite au moins une zone de détection de proximité et/ou ne se trouve pas dans l'au moins une zone de détection de distance.

7. Système selon l'une quelconque des revendications 1 à 6, **caractérisé en ce que**
au moins l'un des appareils de détection est réalisé de sorte à pouvoir émettre des ondes électromagnétiques d'une première bande de fréquence pour la détection du transpondeur (4, 8) et à les recevoir du transpondeur (4, 8), et
au moins l'un des appareils de détection est réalisé de sorte à pouvoir émettre des ondes électromagnétiques d'une seconde bande de fréquence pour la détection du transpondeur (4, 8) et à les recevoir du transpondeur (4, 8).

8. Système selon l'une quelconque des revendications 1 à 7, **caractérisé en ce que**
au moins l'un des appareils de détection est réalisé de sorte à émettre et à recevoir du transpondeur (4, 8) des ondes électromagnétiques selon une première norme de transmission pour la détection du transpondeur (4, 8), et
au moins l'un des appareils de reconnaissance est réalisé de sorte à émettre et à recevoir du transpondeur (4, 8) des ondes électromagnétiques selon une deuxième norme de transmission pour la reconnaissance du transpondeur (4, 8)

9. Système selon l'une quelconque des revendications 1 à 8, **caractérisé en ce que** le réseau de surveillance est réalisé pour être exploité selon une troisième norme de transmission.

10. Système selon l'une quelconque des revendications 1 à 9, **caractérisé en ce que** le réseau de surveillance est partiellement ou totalement maillé.

11. Système selon l'une quelconque des revendications 1 à 10, **caractérisé en ce qu'**au moins un dispositif de surveillance (28 ; 30) est agencé dans un conteneur de produits stériles (24 ; 26).

12. Système selon l'une quelconque des revendications 1 à 11, **caractérisé en ce que**
au moins un dispositif de surveillance (27) est agencé dans une zone de stockage d'un environnement opératoire,
au moins un dispositif de surveillance (32, 34) est agencé dans l'environnement opératoire dans une zone stérile (12) sur une table de desserte (20, 22) dans l'environnement d'une table d'opération (10) et
au moins un dispositif de surveillance (36) est agencé dans l'environnement opératoire dans la zone stérile (12) sur la table d'opération (10).

13. Système selon l'une quelconque des revendications 1 à 12, **caractérisé par** un système de positionnement qui est réalisé de sorte à pouvoir détecter et/ou enregistrer des positions relatives des au moins deux dispositifs de surveillance (27-36).

14. Procédé de surveillance d'au moins un produit médical (2, 6) dans un environnement opératoire, dans lequel au moins deux dispositifs de surveillance (27-36) reliés ou pouvant être reliés l'un à l'autre par conduction de données dans un réseau de surveillance sont agencés ou peuvent être agencés de sorte que des zones de détection (39-48) des au moins deux dispositifs de surveillance (27-36) s'étendant autour du dispositif de surveillance respectif (27-36) se chevauchent, dans lequel le produit médical (2,6) est en particulier réutilisable et/ou un instrument chirurgical, avec les étapes suivantes consistant à
- détecter si un transpondeur (4, 8) du produit médical (2, 6) se trouve dans l'une des zones de détection (39-48), et
- mémoriser des moments de détection du transpondeur (4, 8),
**caractérisé par**
- calculer des durées pendant lesquelles le transpondeur (4, 8) se trouve exclusivement dans l'une des zones de détection (39-48) des au moins deux dispositifs de surveillance (27-36), comparer les durées calculées à des durées de consigne correspondantes mémorisées et émettre des signaux d'alarme correspondants en cas de dépassement de durées de consigne et/ou
- enregistrer des séquences dans lesquelles les au moins deux dispositifs de surveillance (27-36) détectent le transpondeur (4, 8) dans les zones de détection (39-48), comparer les séquences enregistrées à des séquences de consigne correspondantes mémorisées et émettre des signaux d'alarme correspondants en cas d'écart par rapport aux séquences de consigne.
